# EUROPEAN PATENT APPLICATION

(11) **EP 4 671 375 A1**
(43) Date of publication of application: **31.12.2025**
(21) Application number: 24819657.8
(22) Date of filing: 07.06.2024
(51) Int. Cl.: C12N 15/81, C12P 23/00, C07K 14/39

(54) **YARROWIA SP. MICROORGANISM FOR PRODUCING RETINOID WITH INCREASED PHO84 PROTEIN ACTIVITY AND RETINOID PRODUCTION METHOD USING SAME**

(30) Priority: 07.06.2023 KR 20230073122
(71) Applicant: CJ Cheiljedang Corporation, Seoul 04560 (KR)
(72) Inventor: LEE, Dong Pil, Seoul 04560 (KR); PARK, Hye Min, Seoul 04560 (KR); KIM, Jae Eung, Seoul 04560 (KR); LEE, Peter, Seoul 04560 (KR)
(74) Representative: Meissner Bolte Partnerschaft mbB
(86) International application number: PCT/KR2024/095874
(87) International publication number: WO 2024/253507

(57) **Abstract**

The present application relates to: a *Yarrowia* sp. microorganism with retinoid production ability, in which the activity of the PHO84 protein is increased compared to the endogenous activity thereof; a retinoid production method using same; a method for producing a *Yarrowia* sp. microorganism with retinoid production ability; and a composition for producing a retinoid.

## Description

### [Technical Field]

The present disclosure relates to a *Yarrowia* sp. microorganism having retinoid-producing ability in which the activity of a PHO84 protein is increased compared to its endogenous activity; a method for producing retinoid using the same; a method for preparing a *Yarrowia* sp. microorganism having retinoid-producing ability; and a composition for producing retinoid.

### [Background Art]

Retinol, a fat-soluble vitamin, is an essential vitamin involved in eye health for improving night blindness, strengthening immunity, skin health, and the like. Currently, it is produced and marketed mainly by leading global companies through chemical synthesis, but research is being conducted to produce retinol based on microbial fermentation.

To this end, although numerous technologies have been developed for stabilizing the retinol compound itself in a composition or product containing retinol (US 6858217 B2), methods for stably increasing retinol production have been only minimally developed.

### [Disclosure]

### [Technical Problem]

A problem to be solved by the present disclosure is to provide a *Yarrowia* sp. microorganism having retinoid-producing ability in which the activity of the PHO84 protein is increased compared to its endogenous activity; a method for producing retinoid using the same; a method for preparing a *Yarrowia* sp. microorganism having retinoid-producing ability; and a composition for producing retinoid.

### [Technical Solution]

An object of the present disclosure is to provide a *Yarrowia* sp. microorganism having retinoid-producing ability in which the activity of a PHO84 protein is increased compared to its endogenous activity.

Another object of the present disclosure is to provide a method for producing retinoid, comprising culturing the microorganism of the present disclosure in a medium.

Still another object of the present disclosure is to provide a method for preparing a *Yarrowia* sp. microorganism having retinoid-producing ability, comprising increasing the activity of a PHO84 protein in the *Yarrowia* sp. microorganism having retinoid-producing ability.

Yet another object of the present disclosure is to provide a composition for producing retinoid, comprising one or more of the microorganism of the present disclosure and a culture thereof.

Yet another object of the present disclosure is to provide a use of the microorganism of the present disclosure or a culture thereof for retinoid production.

### [Advantageous Effects]

Retinoid can be produced using the microorganism of the present disclosure.

### [Brief Description of the Drawings]

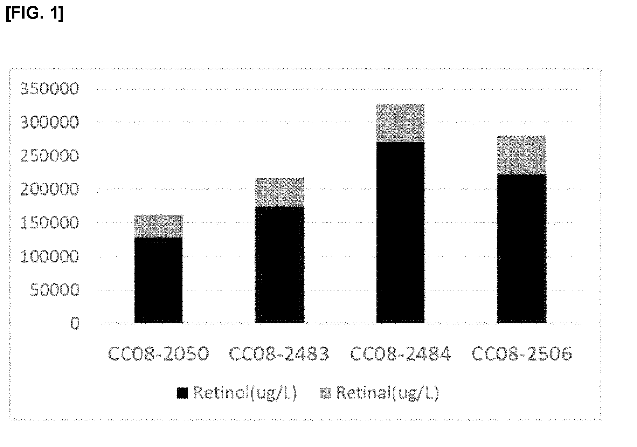

FIG. 1 is a diagram illustrating the retinoid-producing ability of strains.

### [Detailed Description of Preferred Embodiments]

The present disclosure will be described in detail as follows. Meanwhile, each description and embodiment disclosed in the present disclosure may also be applied to other descriptions and embodiments. That is, all combinations of various elements disclosed in the present disclosure fall within the scope of the present disclosure. Further, the scope of the present disclosure is not limited by the specific description described below. Furthermore, a number of papers and patent documents are referenced and cited throughout the present specification. The disclosures of the cited papers and patent documents are incorporated herein by reference in their entirety to further clarify the level of art and the description of the present disclosure.

One aspect of the present disclosure provides a *Yarrowia* sp. microorganism having retinoid-producing ability in which the activity of a PHO84 (high-affinity inorganic phosphate (Pi) transporter PHO84) protein is increased compared to its endogenous activity.

The increased PHO84 protein activity may be measured by measuring the amount of PHO84 protein or polynucleotide encoding the same, or by measuring the retinoid-producing ability (or yield), but the measurement is not limited thereto. For example, in the case where the retinoid-producing ability of the microorganism of the present disclosure is increased compared to that of a natural wild-type *Yarrowia* sp. microorganism or a non-modified *Yarrowia* sp. microorganism (*e.g.,* a *Yarrowia* sp. microorganism expressing a wild-type polypeptide (*e.g.,* the polypeptide of SEQ ID NO: 1) having a wild-type PHO84 protein activity), the increased PHO84 protein activity may be measured by measuring the increased retinoid-producing ability, but the measurement is not limited thereto.

The "PHO84 protein (high-affinity inorganic phosphate (Pi) transporter PHO84)" of the present disclosure is a type of inorganic phosphate transporter, and may function both as a high-affinity inorganic phosphate (Pi) transporter and a low-affinity manganese transporter.

The PHO84 protein of the present disclosure may comprise one or more proteins of YALI0A15125, YALI0A21307, and YALI0B19008. The proteins YALI0A15125, YALI0A21307, and YALI0B19008 may be used interchangeably with YALI0A15125p, YALI0A21307p, and YALI0B19008p, respectively.

The PHO84 protein of the present disclosure may comprise any PHO84 protein that is capable of increasing retinoid-producing ability.

In one embodiment, the PHO84 protein of the present disclosure may have an increased activity compared to an endogenous or wild-type PHO84 protein in a *Yarrowia* sp. microorganism, thereby increasing the retinoid-producing ability of the microorganism.

In one embodiment, the PHO84 protein of the present disclosure may comprise, have, consist of, or essentially consist of an amino acid sequence having 80% or more homology or identity to one or more amino acid sequences of SEQ ID NO: 1, SEQ ID NO: 3, and SEQ ID NO: 5.

In one embodiment of the foregoing embodiment, the YALI0A15125 protein (*i.e.,* YALI0A15125p), YALI0A21307 protein (*i.e.,* YALIOA21307p), and YALI0B19008 protein (*i.e.,* YALI0B19008p) among the PHO84 proteins of the present disclosure may comprise, have, consist of, or essentially consist of an amino acid sequence having 80% or more homology or identity to SEQ ID NO: 1, SEQ ID NO: 3, and SEQ ID NO: 5, respectively.

For example, an amino acid sequence of the PHO84 protein of the present disclosure may be encoded by one or more genes of YALI0A15125 (*i.e.*, YALI0A15125g), YALI0A21307 (*i.e.,* YALI0A21307g), and YALIOB19008 (*i.e.,* YALI0B19008g), but is not limited thereto. The amino acid sequence of the PHO84 protein may be obtained from various databases, such as NCBI's GenBank, but is not limited thereto.

In one embodiment, the PHO84 protein of the present disclosure may be derived from *Yarrowia lipolytica,* but is not limited thereto.

Further, although an embodiment of the PHO84 protein of the present disclosure is described as comprising one or more sequences of SEQ ID NO: 1, SEQ ID NO: 3, and SEQ ID NO: 5, this does not exclude the addition of meaningless sequences upstream or downstream one or more amino acid sequences of SEQ ID NO: 1, SEQ ID NO: 3, and SEQ ID NO: 5, naturally occurring mutations, or silent mutations thereof. Further, it is apparent to those skilled in the art that any protein that exhibits an activity identical or corresponding to that of the protein comprising one or more sequences of SEQ ID NO: 1, SEQ ID NO: 3, and SEQ ID NO: 5 corresponds to the PHO84 protein of the present disclosure.

For example, the PHO84 protein of the present disclosure may comprise one or more amino acid sequences of SEQ ID NO: 1, SEQ ID NO: 3, and SEQ ID NO: 5, or comprise, have, consist of, or essentially consist of an amino acid sequence having at least 80% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more homology or identity to one or more amino acid sequences of SEQ ID NO: 1, SEQ ID NO: 3, and SEQ ID NO: 5. Further, it is apparent that, as long as an amino acid sequence has the above homology or identity and exhibits an efficacy corresponding to that of the protein, it falls within the scope of the present disclosure even if part of the sequence has a deletion, modification, substitution, or addition.

Even if the present disclosure describes 'a polypeptide (or a protein) comprising an amino acid sequence represented by a specific SEQ ID NO', 'a polypeptide (or a protein) consisting of an amino acid sequence represented by a specific SEQ ID NO', or 'a polypeptide (or a protein) having an amino acid sequence represented by a specific SEQ ID NO', it is apparent that a protein having an amino acid sequence with deletion, modification, substitution, or addition in part of the sequence may also be used in the present disclosure, as long as it has an activity identical or corresponding to that of the polypeptide or protein consisting of the amino acid sequence of the corresponding SEQ ID NO. For example, this includes cases of addition of a sequence that does not alter the function of the protein to the N-terminus and/or C-terminus of the amino acid sequence, naturally occurring mutations, silent mutations thereof, or conservative substitutions.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of residues. Generally, a conservative substitution may hardly affect or not affect the activity of the proteins.

As used herein, the term "identity" or "homology'" refers to the degree of similarity between two given amino acid sequences or nucleotide sequences and may be expressed as a percentage. The terms "homology" and "identity" may often be used interchangeably in the present disclosure.

The sequence homology or identity of a conserved polynucleotide or polypeptide (including protein) is determined by standard alignment algorithms, and a default gap penalty established by the program used may be used together. Substantially, homologous or identical sequences are generally capable of hybridizing with the whole sequence or a part of the sequence, corresponding to at least about 50%, 60%, 70%, 80%, or 90% of the full length, under moderately or highly stringent conditions. It is apparent that hybridization also comprises hybridization with a polynucleotide containing a general codon or a codon considering codon degeneracy in the polynucleotide.

Whether any two polynucleotide or polypeptide (including protein) sequences have homology, similarity, or identity may be determined by, for example, a known computer algorithm such as the "FASTA" program using default parameters as in Pearson et al. (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, and FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET AL.] (1988) SIAM J Applied Math 48: 1073)). For example, homology, similarity, or identity may be determined using BLAST of the National Center for Biotechnology Information, or ClustalW.

The homology, similarity, or identity between polynucleotides or polypeptides (including proteins) may be determined by, for example, comparing sequence information using a GAP computer program, such as Needleman et al., (1970), J Mol Biol. 48:443, as described in, for example, Smith and Waterman, Adv. Appl. Math (1981) 2:482. Briefly, a GAP program may be defined as the value acquired by dividing the number of similarly aligned symbols (namely, nucleotides or amino acids) by the total number of symbols in the shorter of two sequences. The default parameters for the GAP program may include: (1) a binary comparison matrix (comprising values of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

Further, whether any two polynucleotide or polypeptide (including protein) sequences have homology, similarity, or identity may be confirmed by comparing sequences through Southern hybridization experiments under appropriate hybridization conditions, wherein the appropriate hybridization conditions are within the scope of the technical field and can be determined by methods well known to those skilled in the art (such as J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York), but are not limited thereto.

As used herein, the term "polynucleotide", which is a polymer of nucleotides in which nucleotide monomers are connected in a long chain shape by covalent bonds, is a DNA strand having a certain length or more.

The polynucleotide sequence encoding the PHO84 protein of the present disclosure may be referred to as one or more sequences of the YALI0A15125 gene, YALI0A21307 gene, and YALI0B19008 gene, and may comprise a polynucleotide sequence encoding the amino acid sequence represented by one or more of SEQ ID NO: 1, SEQ ID NO: 3, and SEQ ID NO: 5.

In the polynucleotide, various modifications may be made in the coding region as long as the amino acid sequence is not changed in consideration of codon degeneracy or codons preferred in organisms that are intended to express the polypeptide or protein. Specifically, the polynucleotide may comprise, consist of, or essentially consist of one or more of SEQ ID NO: 2, SEQ ID NO: 4, and SEQ ID NO: 6, or a polynucleotide sequence having 80% or more homology or identity thereto, but is not limited thereto. For example, the polynucleotide may consist of a nucleotide sequence having 80% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more homology or identity to one or more sequences of SEQ ID NO: 2, SEQ ID NO: 4, and SEQ ID NO: 6, or may be a degenerate sequence thereof, but is not limited thereto.

Further, the polynucleotide of the present disclosure may comprise a probe, for example, without limitation as long as it is a sequence capable of hybridizing with a complementary sequence to the entirety or part of the polynucleotide base sequence under stringent conditions.

As used herein, the term "stringent conditions" refers to conditions that enable specific hybridization between polynucleotides. These conditions are specifically described in the literature (such as J. Sambrook *et al., supra*). Examples of stringent conditions may be conditions in which polynucleotides having high homology or identity of 40% or more, specifically 90% or more, more specifically 95% or more, 96% or more, 97% or more, or 98% or more, or even more specifically 99% or more, hybridize with each other, but polynucleotides having low homology or identity do not hybridize with each other; or washing conditions of typical Southern hybridization, *i.e.,* conditions of washing once, or specifically two to three times, at a salt concentration and temperature corresponding to 1×SSC, 0.1% SDS at 60°C, specifically 0.1×SSC, 0.1% SDS at 60°C, or more specifically 0.1×SSC, 0.1 % SDS at 68°C.

The hybridization may occur between nucleotides having complementary base sequences, but the hybridized polynucleotides may include some base mismatches depending on the stringency of the hybridization. The term "complementary" is used to describe the relationship between nucleotide bases capable of hybridizing with each other. For example, with regard to DNA, adenine is complementary to thymine, and cytosine is complementary to guanine. Hence, the polynucleotide of the present disclosure may also include substantially similar nucleic acid sequences as well as isolated nucleic acid fragments that are complementary to the entire sequence.

Specifically, polynucleotides having homology or identity may be detected by employing hybridization conditions comprising a hybridization step at a Tm of 55°C and utilizing the above-described conditions. In addition, the Tm value may be 60°C, 63°C, or 65°C, but is not limited thereto, and may be appropriately adjusted by those skilled in the art.

The appropriate stringency for hybridizing a polynucleotide depends on the length and degree of complementarity of the polynucleotide, and the variables are well known in the relevant technical field (see J. Sambrook *et al., supra*).

For example, homologous or identical polynucleotide sequences can generally hybridize under stringent conditions along the whole sequence or at least about 50%, 60%, 70%, 80%, or 90% of the full length.

The "vector" of the present disclosure refers to a DNA construct for delivering a desired polynucleotide into a suitable host or host cell. For example, it may comprise a base sequence of a polynucleotide encoding a desired polypeptide or protein that is operably linked to a suitable expression regulatory region (or expression control sequence) such that the desired polypeptide or protein may be expressed in a suitable host, but is not limited thereto.

The expression regulatory region may comprise a promoter capable of initiating transcription, any operator sequence for controlling such transcription, a sequence encoding a suitable mRNA ribosome binding site, and a sequence controlling termination of transcription and translation. The vector, after being transformed into a suitable host cell (microorganism), may replicate or function independently of the host genome, or may be integrated into the genome itself to replicate or function.

The vector of the present disclosure may be an insertion vector for inserting a polynucleotide for increasing the activity of the PHO84 protein of the present disclosure into a chromosome, but is not limited thereto. The insertion of the polynucleotide into the chromosome may be performed by any method known in the art, for example, homologous recombination, but is not limited thereto. The vector may further comprise a selection marker to confirm transformation into a host cell, or further, insertion into the chromosome of the host cell. The selection marker is for selecting cells transformed with vectors, or for confirming the chromosomal insertion of a desired polynucleotide, and markers that confer selectable phenotypes such as drug resistance, auxotrophy, resistance to cytotoxic agents, or expression of surface polypeptides or proteins may be used. In an environment treated with a selective agent, only cells expressing the selection marker survive or exhibit other phenotypic traits, thereby enabling selection of transformed cells. The insertion vector may not comprise an origin of replication required for replication in transformed cells.

The vector used in the present disclosure is not particularly limited, and any vector known in the art may be used. Examples of commonly used vectors comprise natural or recombinant plasmids, cosmids, viruses, and bacteriophages. For example, pWE15, M13, MBL3, MBL4, IXII,ASHII,APII, t10, t11, Charon4A, Charon21A, *etc.* may be used as a phage vector or a cosmid vector, and pDZ system, pBR system, pUC system, pBluescript II system, pGEM system, pTZ system, pCL system, pET system, *etc.* may be used as a plasmid vector. Specifically, pDZ, pDC, pDCM2, pACYC177, pACYC184, pCL, pECCG117, pUC19, pBR322, pMW118, pCC1BAC vectors, *etc.* may be used.

As used herein, the term "transformation" refers to the introduction of a desired polynucleotide and/or a vector comprising the same into a host cell (microorganism), thereby altering the genetic traits of the host cell (microorganism). In the present disclosure, transformation may mean introducing a vector comprising a polynucleotide for increasing the activity of a PHO84 protein into a host cell, thereby altering the genetic traits of the host cell. The transformed polynucleotide may be inserted into a chromosome of the host cell or located outside the chromosome. Further, the polynucleotide may comprise a DNA and/or RNA encoding a protein of interest (*e.g.,* PHO84 protein). The polynucleotide may be introduced in a suitable form depending on the purpose of introduction. For example, a polynucleotide for the expression of a protein of interest may be introduced into a host cell in the form of an expression cassette, which is a gene construct comprising all elements required for self-expression. The expression cassette may usually comprise a promoter operably linked to the coding sequence of the desired polynucleotide, a transcription termination signal, a ribosome binding site, and a translation termination signal. The expression cassette may be in the form of an expression vector capable of self-replication. Further, the polynucleotide may be introduced into a host cell in the form *per se* and be operably linked to a sequence required for expression in the host cell, but is not limited thereto.

As used herein, the term "operably linked" refers to a constitution in which a regulatory sequence is positioned appropriately to control the expression of a coding sequence. Hence, "operably linked" includes a regulatory region of a functional domain with a known or desired activity, such as a promoter, terminator, signal sequence, or enhancer region, being attached or linked to a target (gene or polypeptide) to regulate its expression, secretion, or function in accordance with the known or desired activity. For example, it may mean that a polynucleotide sequence encoding a polypeptide is functionally linked to a promoter sequence that initiates and mediates transcription of the polynucleotide.

The method of transforming a vector of the present disclosure comprises any method of introducing a nucleic acid into a cell, and may be performed by selecting a suitable standard technique known in the art depending on the host cell. For example, methods such as electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method may be used, but are not limited thereto.

As used herein, the term "microorganism (or strain)" includes both wild-type microorganisms and prokaryotic or eukaryotic microorganisms that have undergone genetic modification naturally or artificially. It may be a microorganism in which a specific mechanism is weakened or enhanced due to insertion of a foreign gene or enhancement or inactivation of activity of an endogenous gene, and may be a microorganism comprising a genetic modification for production of a polypeptide, protein, or product of interest. In the present disclosure, the terms "microorganism", "strain", "host", and "host cell" may be used interchangeably.

As used herein, the term "recombinant microorganism" refers to a microorganism that has been genetically modified to exhibit a different genotype and/or phenotype compared to a naturally occurring microorganism (*e.g.,* when the genetic modifications have an effect on a coding nucleic acid sequence of a microorganism), and may include all descendants or potential descendants of the microorganism. In the present disclosure, the terms "recombinant microorganism", "genetically modified microorganism", "recombinant host cell", "recombinant cell", and "recombinant strain" may be used interchangeably. The recombinant microorganism may, for example, express a gene that is not found in its natural (non-recombinant) form, not express a gene that is expressed in its natural form, or express a natural gene in a manner different from that in which it is expressed in its natural form.

For example, the microorganism of the present disclosure may be a recombinant microorganism with increased PHO84 protein activity compared to its endogenous activity, but is not limited thereto.

The microorganism of the present disclosure may be a microorganism having retinoid-producing ability. The term "microorganism having retinoid-producing ability" may be used interchangeably with "retinoid-producing microorganism".

The microorganism of the present disclosure may be a microorganism into which a polynucleotide encoding lycopene cyclase/phytoene synthase (crtYB) and phytoene desaturase (crtl) proteins has been introduced so as to enable a microorganism intrinsically lacking retinoid-producing ability to have retinoid-producing ability, or to further increase the retinoid-producing ability of a microorganism having retinoid-producing ability, and thereby exhibit the activities of these proteins or have increased activities of these proteins. The lycopene cyclase/phytoene synthase or phytoene desaturase may be proteins derived from *Xanthophyllomyces dendrorhous,* but are not limited thereto as long as they are proteins that exhibit the same or similar activities. In one embodiment, the lycopene cyclase/phytoene synthase or phytoene desaturase may consist of or comprise the amino acid sequence of SEQ ID NO: 45 or SEQ ID NO: 47, respectively, but may also consist of or comprise an amino acid sequence having at least 60%, 65%, 70%, 75%, 80%, 85%, 86%, 87%, 88%, 89%, 90%, 91%, 92%, 93%, 94%, 95%, 96%, 97%, 98%, or 99% homology or identity to the above amino acid sequences while exhibiting an activity corresponding to the lycopene cyclase/phytoene synthase or phytoene desaturase. Further, it is apparent that a protein with deletion, modification, substitution, or addition to a part thereof is also included in the lycopene cyclase/phytoene synthase or phytoene desaturase, as long as the protein has the aforementioned homology or identity and exhibits an activity corresponding to the lycopene cyclase/phytoene synthase or phytoene desaturase. Further, in one embodiment, the polynucleotide encoding the lycopene cyclase/phytoene synthase or phytoene desaturase may consist of or comprise the sequence of SEQ ID NO: 46 or SEQ ID NO: 48, respectively. In the polynucleotide, various modifications may be made in the coding region as long as the amino acid sequence is not changed in consideration of codon degeneracy or codons preferred in the microorganism of the present disclosure. Specifically, the polynucleotide may comprise or consist of a base sequence having 60% or more, 70% or more, 80% or more, 85% or more, 90% or more, 95% or more, 96% or more, 97% or more, 98% or more, or 99% or more homology or identity to the sequence of SEQ ID NO: 46 or SEQ ID NO: 48, but is not limited thereto.

Further, the microorganism of the present disclosure may be a microorganism into which a polynucleotide encoding beta-carotene 15,15'-oxygenase (BLH) protein has been introduced so as to enable a microorganism intrinsically lacking retinoid-producing ability to have retinoid-producing ability, or to further increase the retinoid-producing ability of a microorganism having retinoid-producing ability, and thereby exhibit beta-carotene 15,15'-oxygenase activity or have increased beta-carotene 15,15'-oxygenase activity. The beta-carotene 15,15'-oxygenase may be a protein derived from uncultured marine bacterium 66A03, but is not limited thereto as long as it is a protein that exhibits the same or similar activity. In one embodiment, the beta-carotene 15,15'-oxygenase may consist of or comprise the amino acid sequence of SEQ ID NO: 49 but may also consist of or comprise an amino acid sequence having at least 60%, 65%, 70%, 75%, 80%, 85%, 86%, 87%, 88%, 89%, 90%, 91%, 92%, 93%, 94%, 95%, 96%, 97%, 98%, or 99% homology or identity to the amino acid sequence of SEQ ID NO: 49 while exhibiting an activity corresponding to the beta-carotene 15,15'-oxygenase. Further, it is apparent that a protein with deletion, modification, substitution, or addition to a part thereof is also included in the beta-carotene 15,15'-oxygenase, as long as the protein has the aforementioned homology or identity and exhibits an activity corresponding to the beta-carotene 15,15'-oxygenase. Further, in one embodiment, the polynucleotide encoding the beta-carotene 15,15'-oxygenase may consist of or comprise the sequence of SEQ ID NO: 50. In the polynucleotide, various modifications may be made in the coding region as long as the amino acid sequence is not changed in consideration of codon degeneracy or codons preferred in the microorganism of the present disclosure. Specifically, the polynucleotide may comprise or consist of a base sequence having 60% or more, 70% or more, 80% or more, 85% or more, 90% or more, 95% or more, 96% or more, 97% or more, 98% or more, or 99% or more homology or identity to the sequence of SEQ ID NO: 50, but is not limited thereto.

The CC08-2050 microorganism (identical to KCCM13294P) of the present disclosure may comprise: a lycopene cyclase/phytoene synthase comprising the amino acid sequence of SEQ ID NO: 45 and/or a polynucleotide encoding the same; a phytoene desaturase comprising the amino acid sequence of SEQ ID NO: 47 and/or a polynucleotide encoding the same; and a beta-carotene 15,15'-oxygenase comprising the amino acid sequence of SEQ ID NO: 49 and/or a polynucleotide encoding the same.

In one embodiment, the microorganism of the present disclosure may be a microorganism with increased retinoid-producing ability as a result of increased activity of a PHO84 protein.

The microorganism of the present disclosure may be a microorganism that naturally has a PHO84 protein or retinoid-producing ability, or a parent strain having a PHO84 protein or retinoid-producing ability, in which the PHO84 protein of the present disclosure is further increased.

In one example, the microorganism of the present disclosure may comprise all microorganisms with increased PHO84 protein activity of the present disclosure that are capable of producing retinoids.

For example, the microorganism of the present disclosure may be a recombinant strain with increased retinoid-producing ability as a result of increased activity of the PHO84 protein of the present disclosure in a natural wild-type microorganism, a microorganism having retinoid-producing ability, and/or a microorganism comprising a PHO84 protein. The recombinant strain with increased retinoid-producing ability may be a microorganism with increased retinoid-producing ability compared to a natural wild-type microorganism or a microorganism in which the activity of the PHO84 protein of the present disclosure has not been increased, but is not limited thereto.

For example, the microorganism in which the PHO84 protein of the present disclosure has not been increased, used as a reference strain for comparing whether retinoid-producing ability has increased, may be CC08-2050 (KCCM13294P, Ref. Park et al., Metabolic Engineering 2022;73:26-37), but is not limited thereto. The deposited strain name of the CJ2050 strain disclosed in the cited reference (Park et al., Metabolic Engineering 2022;73:26-37) is the CC08-2050 strain of the present disclosure, and thus, the CC08-2050 strain of the present disclosure and the CJ2050 strain are the same strain.

In one example, the recombinant strain and microorganism with increased retinoid-producing ability may have an increased retinoid-producing ability compared to the retinoid-producing ability of the parent strain before modification or a non-modified microorganism by about 1% or more, about 2% or more, about 5% or more, about 10% or more, about 20% or more, or about 30% or more (the upper limit is not particularly limited and may be, for example, about 200% or less), and in another example, by about 1.01-fold or more, about 1.02-fold or more, about 1.05-fold or more, about 1.07-fold or more, about 1.1-fold or more, about 1.2-fold or more, or about 1.3-fold or more (the upper limit is not particularly limited and may be, for example, about 10-fold or less), but is not limited thereto as long as there is a positive increase compared to the producing ability of the parent strain before modification or the non-modified microorganism. The term "about" encompasses a range of ±0.5, ±0.4, ±0.3, ±0.2, ±0.1, *etc*., and all values equivalent to or similar to the numerical value following the term "about", but is not limited thereto.

As used herein, the term "non-modified microorganism (strain)" does not exclude a microorganism (strain) comprising mutations that may occur naturally, and may refer to a wild-type microorganism (strain) or a natural microorganism (strain) per se, or a microorganism (strain) before a trait is altered by genetic variation due to natural or artificial factors. For example, the non-modified microorganism may refer to a strain in which the activity of the PHO84 protein of the present disclosure is not increased or has not yet been increased. The term "non-modified microorganism" may be used interchangeably with "microorganism (strain) before modification", "non-mutated microorganism (strain)", "parent strain", "parent microorganism", "wild-type microorganism (strain)", or "reference microorganism (strain)".

The microorganism of the present disclosure may be a *Yarrowia* sp. microorganism, but is not limited thereto.

In one embodiment, the *Yarrowia* sp. microorganism of the present disclosure may be *Yarrowia lipolytica,* but is not limited thereto.

As used herein, an "increase" in protein (polypeptide) activity means that the activity of a protein (polypeptide) is increased compared to its endogenous activity in a host cell (microorganism). The increase may be used interchangeably with terms such as "activation", "up-regulation", "overexpression", and "enhancement".

The host cell (microorganism) may be a prokaryotic or eukaryotic microorganism.

The increase in protein (polypeptide) activity may include a host cell (microorganism) both exhibiting a protein (polypeptide) activity that it did not intrinsically possess, or exhibiting an improved protein (polypeptide) activity compared to its endogenous activity or activity before modification.

For example, the "exhibiting a protein (polypeptide) activity that it did not intrinsically possess" or "exhibiting an improved protein (polypeptide) activity" may be due to "introduction of a protein (polypeptide)", but is not limited thereto. The introduction of a protein (polypeptide) may be due to introduction of a gene encoding the protein (polypeptide) into a host cell (microorganism). For example, a polynucleotide encoding a specific protein (polypeptide) may be introduced into a chromosome of a host cell (microorganism), or a vector comprising the polynucleotide encoding a specific protein (polypeptide) may be introduced into the host cell (microorganism), thereby exhibiting or enhancing the activity thereof.

The "endogenous activity" refers to the activity of a specific protein (polypeptide) originally possessed by a host cell (microorganism) before transformation or a non-modified host cell (microorganism), when the trait is altered by genetic variation due to natural or artificial factors. This term may be used interchangeably with "activity before modification".

An increase in activity of a protein (polypeptide) compared to its endogenous activity means that the activity and/or concentration (expression level) of the protein (polypeptide) in a host cell (microorganism) is enhanced compared to the activity and/or concentration (expression level) of the protein (polypeptide) originally possessed by the host cell (microorganism) before modification or a non-modified host cell (microorganism).

In one example, the increase may refer to an emergence of activity of the corresponding protein (polypeptide) that was absent, or an increase in its activity or concentration, generally by about 1% or more, about 10% or more, about 25% or more, about 50% or more, about 75% or more, about 100% or more, about 150% or more, about 200% or more, about 300% or more, about 400% or more, or about 500% or more, up to a maximum of about 1000% or about 2000% or more, relative to the activity or concentration in a host cell (microorganism) before transformation or a non-modified host cell (microorganism), but is not limited thereto.

The increase in activity of a protein (polypeptide) can be achieved by introducing a foreign protein (polypeptide) or by increasing the activity of an endogenous protein (polypeptide). Whether activity of a protein (polypeptide) has increased may be confirmed from the degree of the activity of the protein (polypeptide), its expression level, or the amount of product resulting from the activity of the protein (polypeptide).

The increase in the activity of a protein (polypeptide) can be achieved by various methods well known in the art, and is not limited as long as the activity of the desired protein (polypeptide) can be increased compared to that of the host cell (microorganism) before modification. Specifically, genetic engineering and/or protein engineering well known to those skilled in the art as routine methods in molecular biology may be used, but the method is not limited thereto (*e.g.,* Sitnicka et al. Functional Analysis of Genes. Advances in Cell Biology. 2010, Vol. 2. 1-16, Sambrook et al. Molecular Cloning 2012, etc.).

Specifically, the increase in the activity of a protein (polypeptide) of the present disclosure may be:
1) increase in the intracellular copy number of a polynucleotide encoding the protein (polypeptide);
2) modification of a gene expression regulatory region on a chromosome encoding the protein (polypeptide) (*e.g.*, introduction of a mutation in the expression regulatory region, replacement with a sequence exhibiting strong expression-inducing activity, or insertion of a sequence exhibiting strong expression-inducing activity);
3) modification of a base sequence encoding a start codon or the 5'-UTR of a gene transcript encoding the protein (polypeptide);
4) modification of an amino acid sequence of the protein (polypeptide) to increase the activity of the protein (polypeptide);
5) modification of a polynucleotide sequence encoding the protein (polypeptide) to increase the activity of the protein (polypeptide) *(e.g.,* modification of a polynucleotide sequence of a gene encoding the protein (polypeptide), such that the gene encodes a protein (polypeptide) modified to have increased activity);
6) introduction of a foreign protein (polypeptide) exhibiting the activity of the protein (polypeptide), or a foreign polynucleotide encoding the same;
7) codon optimization of a polynucleotide encoding the protein (polypeptide);
8) selection and modification or chemical modification of exposed regions of the protein (polypeptide) through an analysis of tertiary structure thereof;
9) regulation of the cellular localization of the protein (polypeptide); or
10) a combination of two or more selected from 1) to 9), but is not particularly limited thereto.

For example,
The 1) increase in the intracellular copy number of a polynucleotide encoding the protein (polypeptide) may involve introducing a polynucleotide encoding the protein (polypeptide) operably linked to an appropriate regulatory sequence in the form of a vector comprising the same into a host cell (microorganism). Alternatively, the increase may involve introducing one copy or two or more copies of the polynucleotide encoding the protein (polypeptide) operably linked to an appropriate regulatory sequence into a chromosome of a host cell (microorganism). The introduction into a chromosome may be performed by introducing into a host cell (microorganism) a vector capable of inserting the polynucleotide into a chromosome of the host cell (microorganism), but is not limited thereto. The vector is as described above. The regulatory sequence may be a native sequence (of the same origin) or a foreign sequence (derived from a different gene) with respect to the polynucleotide sequence encoding the protein, a varied sequence thereof, or a different artificial sequence, and may induce the expression of the polynucleotide in the host cell (microorganism).

The 2) replacement of a gene expression regulatory region (or expression control sequence) on a chromosome encoding the protein (polypeptide) with a sequence exhibiting strong expression-inducing activity may involve, for example, introducing a modification in a sequence by deletion, insertion, substitution, or a combination thereof such that the expression-inducing activity of the expression regulatory region is further enhanced, or a replacement with a sequence exhibiting a stronger expression-inducing activity. The expression regulatory region may comprise, but is not particularly limited to, a promoter, an operator sequence, a sequence encoding a ribosome binding site, a sequence controlling the termination of transcription and translation, *etc.* In one example, it may involve replacing the original promoter with a promoter exhibiting a strong expression-inducing activity, but is not limited thereto.

Examples of known promoters exhibiting strong expression-inducing activity comprise cj1 to cj7 promoters (US 7662943 B2), a lac promoter, a trp promoter, a trc promoter, a tac promoter, a lambda phage PR promoter, a PL promoter, a tet promoter, a gapA promoter, a SPL7 promoter, a SPL13(sm3) promoter (US 10584338 B2), an O2 promoter (US 10273491 B2), a tkt promoter, a yccA promoter, a TEF promoter, *etc.,* but are not limited thereto. In one embodiment, the microorganism of the present disclosure may have a promoter of a gene encoding a PHO84 protein (*e.g.*, YALI0A15125, YALI0A21307, and/or YALI0B19008 protein) replaced by a TEF promoter, but is not limited thereto.

The 3) modification of a base sequence encoding a start codon or 5'-UTR of a gene encoding the protein (polypeptide) may involve, for example, substituting with another start codon with a higher protein (polypeptide) expression rate as compared to an endogenous start codon, or a modification to code for a ribosome binding site (RBS) sequence with a higher protein (polypeptide) expression rate as compared to an endogenous RBS sequence, but is not limited thereto.

The 4) and 5) modification of the amino acid sequence or the polynucleotide sequence of the protein (polypeptide) may involve introducing a modification in the sequence by deletion, insertion, substitution, or a combination thereof in the amino acid sequence of the protein (polypeptide) or the polynucleotide sequence encoding the protein (polypeptide), or replacing with an amino acid sequence or polynucleotide sequence modified to exhibit increased activity, but is not limited thereto. The sequence modification may, for example, be performed by inserting a polynucleotide with a modified sequence into a chromosome through homologous recombination, but is not limited thereto.

The 6) introduction of a foreign polynucleotide exhibiting the activity of the protein (polypeptide) may involve introducing a foreign polynucleotide into a host cell (microorganism), the foreign polynucleotide encoding a protein (polypeptide) exhibiting an activity identical or similar to that of the protein (polypeptide). The foreign polynucleotide is not limited in its origin or sequence as long as it exhibits an activity identical or similar to that of the protein (polypeptide). The method used for the introduction may be performed by appropriately selecting a known transformation method by those skilled in the art. As the introduced polynucleotide is expressed in the host cell, the protein (polypeptide) may be produced, and the activity thereof may be increased.

The 7) codon optimization of a polynucleotide encoding the protein (polypeptide) may be performed such that the transcription or translation of an endogenous polynucleotide is increased in a host cell (microorganism), or such that the transcription or translation of a foreign polynucleotide is optimized in a host cell (microorganism).

The 8) selection and modification or chemical modification of exposed regions of the protein (polypeptide) through an analysis of tertiary structure thereof may involve, for example, determining a template protein candidate based on the degree of similarity of the sequence by comparing the sequence information of a protein (polypeptide) to be analyzed with a database storing the sequence information of known proteins, confirming the structure based thereon, selecting an exposed site to be modified or chemically modified, and modifying or chemically modifying the same.

The 9) regulation of the cellular localization of the protein (polypeptide) may involve targeting the protein (polypeptide) to a specific cellular organelle or a specific intracellular space. For example, it may involve targeting a protein (polypeptide) to the periplasm or cytoplasm by adding or removing a leader sequence that functions in the targeting of the protein (polypeptide), but is not limited thereto.

Such increase in the protein (polypeptide) activity may result in an increase in the activity or concentration of the corresponding protein (polypeptide) as compared to the activity or concentration of the protein (polypeptide) expressed in a wild-type host cell (microorganism) or a host cell (microorganism) before modification, but is not limited thereto.

The modification of a part or the entirety of a polynucleotide in the host cell (microorganism) of the present disclosure may be induced by: (a) a method using homologous recombination using a vector for chromosomal insertion or genome editing using an engineered nuclease (*e.g.*, CRISPR-Cas9), and/or (b) treatment with light, such as ultraviolet light and radiation, and/or chemicals, but is not limited thereto.

In the present disclosure, "retinoid" refers to a group of compounds chemically belonging to the vitamin A family or chemically related thereto.

In one embodiment, the retinoid may be any one selected from the group consisting of retinol, retinal, retinoic acid, and retinyl ester, but is not limited thereto.

In one embodiment, retinol can be converted into other retinoid compounds (*e.g.*, retinal, retinoic acid, and retinyl ester) by methods known in the art.

Another aspect of the present disclosure provides a method for producing retinoid, comprising culturing a *Yarrowia* sp. microorganism having retinoid-producing ability in which the activity of a PHO84 protein is increased compared to its endogenous activity, in medium.

The microorganism is as described in another aspect.

As used herein, the term "cultivation" means growing the microorganism of the present disclosure under appropriately controlled environmental conditions. The cultivation process of the present disclosure may be performed according to a suitable medium and cultivation conditions known in the art. Such a cultivation process may be easily adjusted and used by those skilled in the art according to the selected strain. Specifically, the cultivation may be a batch type, continuous type, and/or fed-batch type, but is not limited thereto.

The microorganism of the present disclosure may be cultured in a common medium containing appropriate carbon sources, nitrogen sources, phosphorus sources, inorganic compounds, amino acids and/or vitamins, *etc.,* while controlling the temperature, pH, *etc.* under aerobic conditions.

In the present disclosure, the carbon sources may comprise carbohydrates such as glucose, saccharose, lactose, fructose, sucrose, maltose, *etc.;* sugar alcohols such as mannitol, sorbitol, *etc.;* organic acids such as pyruvic acid, lactic acid, citric acid, *etc.;* and amino acids such as glutamic acid, methionine, lysine, *etc.* Further, natural organic nutrient sources such as starch hydrolysate, molasses, blackstrap molasses, rice bran, cassava, sugarcane residue, and corn steep liquor may be used. Specifically, carbohydrates such as glucose and sterilized pretreated molasses (*i.e.*, molasses converted to reducing sugar) may be used, and appropriate amounts of other carbon sources may be used in various manners without limitation. These carbon sources may be used alone or in combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extracts, yeast extracts, malt extracts, corn steep liquor, casein hydrolysates, fish or decomposition products thereof, defatted soybean cake or degradation products thereof, *etc.* may be used. These nitrogen sources may be used alone or in a combination of two or more thereof, but are not limited thereto.

The phosphorus sources may comprise monopotassium phosphate, dipotassium phosphate, or sodium-containing salts corresponding thereto. As for inorganic compounds, sodium chloride, calcium chloride, iron chloride, magnesium sulfate, iron sulfate, manganese sulfate, calcium carbonate, *etc*. may be used, and in addition, amino acids, vitamins, and/or suitable precursors may be included. These constituents or precursors may be added to the medium in a batch or continuous manner, but are not limited thereto.

During the cultivation of the microorganism of the present disclosure, the pH of the medium may be adjusted by adding compounds such as ammonium hydroxide, potassium hydroxide, ammonia, phosphoric acid, sulfuric acid, *etc.* to the medium in a suitable manner. Further, during the cultivation, foaming may be suppressed by using an antifoaming agent such as fatty acid polyglycol ester. Additionally, oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but are not limited thereto.

Further, the culture medium may contain metal salts such as magnesium sulfate or iron sulfate necessary for growth. Lastly, in addition to the aforementioned substances, essential growth substances such as amino acids and vitamins may be used. Additionally, suitable precursors may be used in the culture medium. The aforementioned raw materials may be added to the culture in a batch or continuous manner by a suitable method during the cultivation process, but are not limited thereto.

During the cultivation of the microorganism of the present disclosure, the pH of the culture may be adjusted by adding compounds such as ammonium hydroxide, potassium hydroxide, ammonia, phosphoric acid, sulfuric acid, *etc.* to the culture in a suitable manner. Further, during the cultivation, foaming may be suppressed by using an antifoaming agent such as fatty acid polyglycol ester. Additionally, oxygen or oxygen-containing gas may be injected into the culture in order to maintain the aerobic state of the culture, or gas may not be injected or nitrogen, hydrogen, or carbon dioxide gas may be injected in order to maintain the anaerobic and microaerobic states, but are not limited thereto.

In the cultivation of the present disclosure, the cultivation temperature may be maintained at 20°C to 35°C, specifically 25°C to 35°C. The cultivation period may be continued until the amount of the useful substance is obtained, and may be about 10 hours to 160 hours, about 20 hours to 130 hours, about 24 hours to 120 hours, about 36 hours to 120 hours, about 48 hours to 120 hours, about 48 hours or more, or about 48 hours, about 72 hours, or about 120 hours, but is not limited thereto.

The method for producing retinoid of the present disclosure may further comprise recovering retinoid from the microorganism or the medium.

The desired retinoid may be recovered from the medium by using an appropriate method known in the art according to the cultivation method of the microorganism of the present disclosure, for example, a batch, continuous, or fed-batch type cultivation method. For example, centrifugation, filtration, treatment with a crystallized protein precipitating agent (salting-out), extraction, sonication, ultrafiltration, dialysis, various types of chromatography such as molecular sieve chromatography (gel filtration), adsorption chromatography, ion exchange chromatography, affinity chromatography, *etc.,* HPLC, and a combination of these methods may be used, but are not limited thereto.

The method for producing retinoid may further comprise a purification process. The purification process may be performed by using an appropriate method known in the art.

The method for producing retinoid of the present disclosure may further comprise conversion of retinol expressed by the microorganism of the present disclosure into a retinoid other than retinol. With regard to the method for producing retinoid of the present disclosure, the conversion may be further included after the cultivation or the recovery. The conversion may be performed by using an appropriate method known in the art. For example, the conversion may be performed using retinol acyltransferase, but is not limited thereto.

In one embodiment, the retinoid other than retinol may be any one selected from the group consisting of retinal, retinoic acid, and retinyl ester, but is not limited thereto as long as it is included in retinoids.

Another aspect of the present disclosure provides a method for preparing a *Yarrowia* sp. microorganism having retinoid-producing ability, comprising increasing the activity of a PHO84 protein in the *Yarrowia* sp. microorganism having retinoid-producing ability.

Another aspect of the present disclosure provides a method for increasing retinoid production, comprising increasing the activity of a PHO84 protein in the *Yarrowia* sp. microorganism having retinoid-producing ability.

The step of increasing the activity of a PHO84 protein may involve modifying a *Yarrowia* sp. microorganism such that the activity of the PHO84 protein is increased compared to its endogenous activity, as described in other aspects.

Another aspect of the present disclosure provides a composition for producing retinoid comprising one or more of a *Yarrowia* sp. microorganism having retinoid-producing ability in which the activity of a PHO84 protein is increased compared to its endogenous activity, and a culture thereof.

The composition of the present disclosure may further comprise any appropriate excipient that is commonly used in compositions for retinoid production. Such excipients may be, for example, a preservative, a wetting agent, a dispersing agent, a suspending agent, a buffering agent, a stabilizing agent, or an isotonic agent, *etc.,* but are not limited thereto.

The PHO84 protein, the *Yarrowia* sp. microorganism with increased activity thereof, and the retinoid, *etc.,* are as described in other aspects.

Another aspect of the present disclosure provides a use of a *Yarrowia* sp. microorganism in which the activity of a PHO84 protein is increased compared to its endogenous activity or a culture thereof for retinoid production.

The PHO84 protein, the *Yarrowia* sp. microorganism with increased activity thereof, and the retinoid, *etc.,* are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1: Preparation of a retinoid-producing Yarrowia sp. strain with increased PHO84 (YALIOA15125) activity

Among the three PHO84 genes, YALI0A15125, YALI0A21307, and YALI0B19008 genes, identified in the *Yarrowia* strain KCCM13294P (CC08-2050, Park et al., Metab Eng. 2022, Jun 6;73:26-37) that produces retinoids, the promoter of the YALI0A15125 gene was replaced with a TEF promoter, an enhanced promoter, to increase the expression of the YALI0A15125 gene. In particular, the sequence of the TEF promoter corresponds to SEQ ID NO: 7.

To this end, the ORF sequence of the YALIOA15125 gene (SEQ ID NO: 2) was obtained based on the nucleotide sequence registered in KEGG (Kyoto Encyclopedia of Genes and Genomes). Additionally, a promoter replacement cassette was constructed using the URA3 gene (SEQ ID NO: 8) of *Y. lipolytica* as a selection marker. At this time, the genomic DNA of CC08-2050 was used as a template and PCR was performed using the primers of SEQ ID NO: 9 and SEQ ID NO: 10, SEQ ID NO: 11 and SEQ ID NO: 12, SEQ ID NO: 13 and SEQ ID NO: 14, SEQ ID NO: 15 and SEQ ID NO: 16, and SEQ ID NO: 17 and SEQ ID NO: 18 on the left homologous region, TEF promoter, URA3, repeat region, and right homologous region fragments, respectively, as shown in Table 1. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR. The cassette thus constructed was introduced into the CC08-2050 strain using the heat shock method (D.-C. Chen et al., Appl Microbiol Biotechnol, 1997), and colonies formed on a solid medium (YLMM1) lacking uracil were obtained. The insertion of the cassette into the genome was confirmed using primers of SEQ ID NO: 19 and SEQ ID NO: 20. The obtained colonies were cultured on 5-FOA solid medium at 30°C for 3 days, and by obtaining colonies grown on the 5-FOA solid medium, the URA3 marker was recovered. The final YALI0A15125 activity-enhanced strain thus obtained was named CC08-2483.

**[Table 1]**

| SEQ ID NO | Sequence (5'- 3') | PCR Product |
|---|---|---|
| 9 | GTCAGCGAAAGGGAGATGAG | Homology left arm |
| 10 | CGCCGCCAACCCGGTCTCTTGAAGAGTTGGAGTGGTTG TGC | |
| 11 | GCACAACCACTCCAACTCTTCAAGAGACCGGGTTGGCG GCG | TEF promoter |
| 12 | GACGAGTCAGACAGGAGGCATTTGAATGATTCTTATAC TC | |
| 13 | GACGAGTCAGACAGGAGGCATTTGAATGATTCTTATAC TC | URA3 |
| 14 | GCGCCGCCAACCCGGTCTCTTGGTGGTATTGTGACTGG GG | |
| 15 | CCCCAGTCACAATACCACCAAGAGACCGGGTTGGCGG CGC | Repeat region |
| 16 | GGTCACGTTGTTATCGGCCATTTTGAATGATTCTTATAC TCAGAAGG | |
| 17 | CCTTCTGAGTATAAGAATCATTCAAAATGGCCGATAACA ACGTGACC | Homology right arm |
| 18 | GAGAATCTTTCCGTGCTTCCAC | |
| 19 | CGTGCATTCCTGGTGCTACG | Check forward |
| 20 | GACAAGAATCAGGTTACCGATG | Check reverse |

The aforementioned YLMM1 and 5-FOA media used had the following compositions:

### <Yarrowia lipolytica Minimal Medium 1 (YLMM1)>

Glucose 20 g/L, Yeast nitrogen base without amino acids 6.7 g/L, Yeast Synthetic Drop-out Medium Supplements without uracil 2 g/L, Agar 15 g/L

### <5-Fluoroorotic Acid (5-FOA) Medium>

Glucose 20 g/L, Yeast nitrogen base without amino acids 6.7 g/L, Yeast Synthetic Drop-out Medium Supplements without uracil 2 g/L, Uracil 50 µg/mL, 5-Fluoroorotic acid (5-FOA) 1 g/L, Agar 15 g/L

### Example 2: Preparation of a retinoid-producing Yarrowia sp. strain with increased PHO84 (YALIOA21307) activity

Among the three PHO84 genes, YALI0A15125, YALI0A21307, and YALI0B19008, identified in the Yarrowia strain KCCM13294P that produces retinoids, the promoter of the YALI0A21307 gene was replaced with a TEF promoter, an enhanced promoter, to increase the expression of the YALI0A21307 gene.

To this end, the ORF sequence of the YALI0A21307 gene (SEQ ID NO: 4) was obtained based on the nucleotide sequence registered in KEGG (Kyoto Encyclopedia of Genes and Genomes). Additionally, a promoter replacement cassette was constructed using the URA3 gene (SEQ ID NO: 8) of *Y. lipolytica* as a selection marker. At this time, the genomic DNA of CC08-2050 was used as a template, and PCR was performed using the primers of SEQ ID NO: 21 and SEQ ID NO: 22, SEQ ID NO: 23 and SEQ ID NO: 24, SEQ ID NO: 25 and SEQ ID NO: 26, SEQ ID NO: 27 and SEQ ID NO: 28, and SEQ ID NO: 29 and SEQ ID NO: 30 on the left homologous region, TEF promoter, URA3, repeat region, and right homologous region fragments, respectively, as shown in Table 2. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR. The cassette thus constructed was introduced into the CC08-2050 strain using the heat shock method (D.-C. Chen et al., Appl Microbiol Biotechnol, 1997), and colonies formed on a solid medium (YLMM1) lacking uracil were obtained. The insertion of the cassette into the genome was confirmed using primers of SEQ ID NO: 31 and SEQ ID NO: 32. The obtained colonies were cultured on 5-FOA solid medium at 30°C for 3 days, and by obtaining colonies grown on the 5-FOA solid medium, the URA3 marker was recovered. The final YALI0A21307 activity-enhanced strain thus obtained was named CC08-2484.

**[Table 2]**

| SEQ ID NO | Sequence (5'- 3') | PCR Product |
|---|---|---|
| 21 | GGTATAGGTGCTTCAATCGCG | Homology left arm |
| 22 | CGCCGCCAACCCGGTCTCTGAATGCCTGAGAGTGGGG TC | |
| 23 | GACCCCACTCTCAGGCATTCAGAGACCGGGTTGGCGG CG | TEF promoter |
| 24 | GACGAGTCAGACAGGAGGCATTTGAATGATTCTTATACT C | |
| 25 | GAGTATAAGAATCATTCAAATGCCTCCTGTCTGACTCGT C | URA3 |
| 26 | GCGCCGCCAACCCGGTCTCTTGGTGGTATTGTGACTGG GG | |
| 27 | CCCCAGTCACAATACCACCAAGAGACCGGGTTGGCGG CGC | Repeat region |
| 28 | CAGTCTTGTTCTGAGCGTCCATTTTGAATGATTCTTATAC TCAGAAGG | |
| 29 | CCTTCTGAGTATAAGAATCATTCAAAATGGACGCTCAGA ACAAGACTG | Homology right arm |
| 30 | GTACCAAGCAGAATCTTTCCG | |
| 31 | CTTAGTTGGGCCACTCTAAC | Check forward |
| 32 | GAATCAGGTTACCGACGGAG | Check reverse |

The aforementioned YLMM1 and 5-FOA media used had the same compositions as described in Example 1.

### Example 3: Preparation of a retinoid-producing Yarrowia sp. strain with increased PHO84 (YALIOB19008) activity

Among the three PHO84 genes, YALI0A15125, YALI0A21307, and YALI0B19008, identified in the *Yarrowia* strain KCCM13294P that produces retinoids, the promoter of the YALI0B19008 gene was replaced with a TEF promoter, an enhanced promoter, to increase the expression of the YALI0B19008 gene.

To this end, the ORF sequence of the YALI0B19008 gene (SEQ ID NO: 6) was obtained based on the nucleotide sequence registered in KEGG (Kyoto Encyclopedia of Genes and Genomes). Additionally, a promoter replacement cassette was constructed using the URA3 gene (SEQ ID NO: 8) of *Y. lipolytica* as a selection marker. At this time, the genomic DNA of CC08-2050 was used as a template and PCR was performed using the primers of SEQ ID NO: 33 and SEQ ID NO: 34, SEQ ID NO: 35 and SEQ ID NO: 36, SEQ ID NO: 37 and SEQ ID NO: 38, SEQ ID NO: 39 and SEQ ID NO: 40, and SEQ ID NO: 41 and SEQ ID NO: 42 on the left homologous region, TEF promoter, URA3, repeat region, and right homologous region fragments, respectively, as shown in Table 3. The PCR conditions consisted of denaturation at 95°C for 1 minute, annealing at 55°C for 1 minute, and polymerization at 72°C for 2 minutes, repeated for 35 cycles. The obtained DNA fragments were assembled into a single cassette through overlap extension PCR. The cassette thus constructed was introduced into the CC08-2050 strain using the heat shock method (D.-C. Chen et al., Appl Microbiol Biotechnol, 1997), and colonies formed on a solid medium (YLMM1) lacking uracil were obtained. The insertion of the cassette into the genome was confirmed using primers of SEQ ID NO: 43 and SEQ ID NO: 44. The obtained colonies were cultured on 5-FOA solid medium at 30°C for 3 days, and by obtaining colonies grown on the 5-FOA solid medium, the URA3 marker was recovered. The final YALI0B19008 activity-enhanced strain thus obtained was named CC08-2506.

**[Table 3]**

| SEQ ID NO | Sequence (5'- 3') | PCR Product |
|---|---|---|
| 33 | CTCCTGACAGGCTGCAAAAG | Homology left arm |
| 34 | CGCCGCCAACCCGGTCTCTCTTTGAGGAACCACACGAC TTTC | |
| 35 | GAAAGTCGTGTGGTTCCTCAAAGAGAGACCGGGTTGG CGGCG | TEF promoter |
| 36 | GACGAGTCAGACAGGAGGCATTTGAATGATTCTTATACT C | |
| 37 | GAGTATAAGAATCATTCAAATGCCTCCTGTCTGACTCGT C | URA3 |
| 38 | GCGCCGCCAACCCGGTCTCTTGGTGGTATTGTGACTGG GG | |
| 39 | CCCCAGTCACAATACCACCAAGAGACCGGGTTGGCGG CGC | Repeat region |
| 40 | CAGAAAGTCGTTTTTTTCCGCCATTTTGAATGATTCTTAT ACTCAGAAGG | |
| 41 | CCTTCTGAGTATAAGAATCATTCAAAATGGCGGAAAAAA ACGACTTTCTG | Homology right arm |
| 42 | GTAGGCGATACCAAGAACCG | |
| 43 | CATCTGTGGCTTGACACTGG | Check forward |
| 44 | GTAGAATCGACCCCATAGAGG | Check reverse |

The aforementioned YLMM1 and 5-FOA media used had the same compositions as described in Example 1.

### Example 4: Comparative evaluation of retinoid-producing ability between expression-enhanced strains of the three PHO84 genes

To compare the retinoid-producing ability of the strains (CC08-2483, CC08-2484, and CC08-2506) constructed in Examples 1 to 3 with that of the control strain (CC08-2050), a flask evaluation was conducted. Each strain was inoculated to an initial OD=2 in a 250 mL corner-baffle flask containing 25 mL of YPDLU medium supplemented with 0.05% BHT (3,5-di-tert-4-butylhydroxytoluene) and 5% Tween 20, and cultured at 30°C and 200 rpm for 48 hours. The YPDLU medium used had the following composition:

### <YPDLU>

Glucose 40 g/L, Bacto peptone 20g/L, Yeast extract 10g/L, Uracil 1g/L, Leucine 1g/L, 1M Phosphate buffer (pH 7.0) 100ml/L

To evaluate the growth level of each strain, OD values were measured at a wavelength of 600 nm using a spectrophotometer. The concentrations of retinol and retinal were quantified by mixing 0.1 mL of the culture broth after completion of culturing with 0.9 mL of acetone containing 4% BHT (Sigma), followed by analysis using HPLC equipment.

The analyzed OD values and retinoid concentrations are as shown in Table 4 below and schematically illustrated in FIG. 1.

**[Table 4]**

| Strain | OD(A₆₀₀) | Retinol (µg/L) | Retinal (µg/L) | Fold change | |
|---|---|---|---|---|---|
| | | | | Retinol | Retinal |
| CC08-2050 | 72 | 129,325 | 33,140 | 1.00 | 1.00 |
| CC08-2483 | 74 | 173,640 | 43,850 | 1.34 | 1.32 |
| CC08-2484 | 77 | 270,030 | 57,110 | 2.09 | 1.72 |
| CC08-2506 | 74 | 223,375 | 56,130 | 1.73 | 1.69 |

Comparing the results of the control strain CC08-2050 and the experimental strains CC08-2483, CC08-2484, and CC08-2506, respectively, in Table 4, the concentrations of both retinol and retinal were increased in the expression-enhanced strains of the three PHO84 genes (CC08-2483, CC08-2484, CC08-2506) compared to the control strain (CC08-2050).

Strain CC08-2483 with increased YALI0A15125 protein activity exhibited a 1.34-fold increase in retinol concentration and a 1.32-fold increase in retinal concentration compared to the control strain. Strain CC08-2484 with increased YALI0A21307 protein activity exhibited a 2.09-fold increase in retinol concentration and a 1.72-fold increase in retinal concentration compared to the control strain. Strain CC08-2506 with increased YALI0B19008 protein activity exhibited a 1.73-fold increase in retinol concentration and a 1.69-fold increase in retinal concentration compared to the control strain.

From the above results, it was confirmed that the retinoid-producing ability of a *Yarrowia* sp. microorganism can be enhanced through increasing the activity of a PHO84 protein.

From the foregoing description, a person skilled in the art to which the present disclosure pertains will be able to understand that the present disclosure may be embodied in other specific forms without modifying the technical concepts or essential characteristics thereof. In this regard, the embodiments described above should be understood to be illustrative rather than restrictive in every respect. The scope of the present disclosure should be construed as the meaning and scope of the appended claims rather than the detailed description, and all changes or variations derived from the equivalent concepts fall within the scope of the present disclosure.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-2050 | Accession number given by the
INTERNATIONAL DEPOSITARY AUTHORITY:
KCCM13294P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 01. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 01. 2022.
¹ Where Rule 6.4(d) applies, such date is the date on which the status of international depositary authority was
acquired.

Form BP/4 (sole page)

한국미생물보존센터 사단법인 한국종균협회
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yurim Bldg., 45, Hongjenae 2ga-gil, Seodaemun-gu, Seoul, 03641, Korea. Tel: 82-2-391-0950, 396-0950 Fax: 82-2-392-2859

## Claims

1. A *Yarrowia* sp. microorganism having retinoid-producing ability in which the activity of a PHO84 (high-affinity inorganic phosphate transporter PHO84) protein is increased compared to its endogenous activity.

2. The microorganism according to claim 1, wherein the PHO84 protein comprises one or more proteins of YALIOA15125, YALI0A21307, and YALI0B19008.

3. The microorganism according to claim 1, wherein the PHO84 protein comprises one or more amino acid sequences of SEQ ID NO: 1, SEQ ID NO: 3, and SEQ ID NO: 5, or an amino acid sequence having 80% or more identity thereto.

4. The microorganism according to claim 1, wherein the PHO84 protein is derived from *Yarrowia lipolytica.*

5. The microorganism according to claim 1, wherein the PHO84 protein is encoded by one or more polynucleotide sequences of SEQ ID NO: 2, SEQ ID NO: 4, and SEQ ID NO: 6, or a polynucleotide sequence having 80% or more identity thereto.

6. The microorganism according to claim 1, wherein the *Yarrowia* sp. microorganism is *Yarrowia lipolytica.*

7. The microorganism according to claim 1, wherein the microorganism has increased retinoid-producing ability compared to a non-modified *Yarrowia* sp. microorganism.

8. The microorganism according to claim 1, wherein the retinoid comprises one or more selected from the group consisting of retinol, retinal, retinoic acid, and retinyl ester.

9. A method for producing retinoid, comprising culturing a *Yarrowia* sp. microorganism having retinoid-producing ability in which the activity of a PHO84 protein is increased compared to its endogenous activity, in a medium.

10. The method according to claim 9, comprising recovering retinoid from the cultured medium or microorganism.

11. The method according to claim 9, wherein the retinoid comprises one or more selected from the group consisting of retinol, retinal, retinoic acid, and retinyl ester.

12. A method for preparing a *Yarrowia* sp. microorganism having retinoid-producing ability, comprising increasing the activity of a PHO84 protein in the *Yarrowia* sp. microorganism having retinoid-producing ability.

13. A composition for producing retinoid, comprising one or more of a *Yarrowia* sp. microorganism having retinoid-producing ability in which the activity of a PHO84 protein is increased compared to its endogenous activity, and a culture thereof.

14. Use of a *Yarrowia* sp. microorganism in which the activity of a PHO84 protein is increased compared to its endogenous activity or a culture thereof for retinoid production.
